# EUROPEAN PATENT APPLICATION

(11) **EP 2 886 051 A1**
(43) Date of publication of application: **24.06.2015**
(21) Application number: 14186894.3
(22) Date of filing: 29.09.2014
(51) Int. Cl.: A61B 5/05, A61B 5/0205, A61B 5/00, G01S 13/02

(54) **Heartbeat measuring apparatus, heartbeat measuring method and driver monitoring system**

(30) Priority: 18.12.2013 KR 20130158473
(71) Applicant: Hyundai Motor Company, Seoul 137-938 (KR); Kia Motors Corporation, Seoul 137-938 (KR); Industry-Academic Cooperation Foundation Yonsei University, Seoul 120-749 (KR)
(72) Inventor: Kim, Tae Wook, 120-823 Seoul (KR); Han, Hong Gul, 612-828 Busan (KR)
(74) Representative: Isarpatent

(57) **Abstract**

A heartbeat measuring apparatus includes a receiver and a measurer. The receiver is configured to receive ultra wideband (UWB) signals penetrating a subject. The measurer is configured to measure a heartbeat of the subject using at least one of center frequency and amplitude of the received UWB signals.

## Description

### TECHNICAL FIELD

The present disclosure relates to a non-contact heartbeat measuring apparatus for measuring a heartbeat without contact a subject, a heartbeat measuring method, and a driver monitoring system for monitoring a driver using the same.

### BACKGROUND

In order to measure a heart state, a method in which electrodes contact a body to measure electrocardiogram has been generally used. However, the method of measuring the electrocardiogram has disadvantage in that the electrodes should be attached to the body and it is difficult for an ordinary person having no expert knowledge to use the method of measuring the electrocardiogram.

In order to solve the above-mentioned problems, a method of measuring a heartbeat using radar reflected from a heart has also been developed. However, the method using the reflected wave, which is based on a change in a distance between a radar transceiver terminal and the heart, may be influenced by a motion of the subject.

### SUMMARY

The present disclosure has been made to solve the above-mentioned problems occurring when using the prior art while advantages achieved by the prior art are maintained intact.

An aspect of the present disclosure provides a heartbeat measuring apparatus capable of measuring a heartbeat regardless of a motion of a subject, a heartbeat measuring method, and a driver monitoring system.

One aspect of the present disclosure relates to a heartbeat measuring apparatus including a receiver and a measurer. The receiver is configured to receive an ultra wideband (UWB) signals penetrating a subject. The measurer is configured to measure a heartbeat of the subject using at least one of center frequency and amplitude of the received UWB signals.

The measurer may include a signal detector configured to detect a UWB signal penetrating a heart of the subject among the received UWB signals, a signal analyzer configured to analyze a variation of at least one of center frequency and amplitude of the detected UWB signal, and a heartbeat measurer configured to measure the heartbeat of the subject by monitoring the variation.

The measurer may include a signal detector configured to detect the UWB signal penetrating the heart of the subject among the received UWB signals, a signal analyzer configured to analyze at least one of center frequency and amplitude of the detected UWB signal, and a heartbeat measurer configured to measure the heartbeat of the subject by monitoring the at least one of the center frequency and amplitude.

The measurer may be configured to determine whether or not a heart of the subject is contracted or relaxed by monitoring the at least one of center frequency and amplitude of the received UWB signals.

Another aspect of the present disclosure encompasses a driver monitoring system including a signal generating apparatus and a heartbeat measuring apparatus. The signal generating apparatus is configured to generate and transmit an ultra wideband (UWB) signal. The heartbeat measuring apparatus is configured to receive the UVB signal penetrating a subject and measure a heartbeat of the subject using at least one of center frequency and amplitude of the received UWB signal.

The heartbeat measuring apparatus may be positioned opposite to the signal generating apparatus with respect to the subject.

The driver monitoring system may further include a breath measuring apparatus configured to receive the UWB signal reflected from the subject and measure a breath or a motion of the subject using a delayed amount of time of the received UWB signal.

The heartbeat measuring apparatus may be configured to determine whether or not the heart of the subject is contracted or relaxed by monitoring the at least one of center frequency and amplitude of the received UWB signal.

Still another aspect of the present disclosure relates to a heartbeat measuring method including receiving ultra wideband (UWB) signals penetrating a subject. A heartbeat of the subject is measured using at least one of center frequency and amplitude of the received UWB signals.

In the measuring of the heartbeat, a UWB signal penetrating a heart of the subject may be detected among the received UWB signals, a variation of at least one of center frequency and amplitude of the detected UWB signal may be analyzed, and the heartbeat of the subject may be measured by monitoring the variation.

In the measuring of the heartbeat, an UWB signal penetrating a heart of the subject may be detected among the received UWB signals, at least one of center frequency and amplitude of the detected UWB signal may be analyzed, and the heartbeat of the subject may be measured by monitoring the at least one of center frequency and amplitude of the detected UWB signal.

In the measuring of the heartbeat of the subject, it may be determined whether or not a heart of the subject is contracted or relaxed by monitoring the at least one of center frequency and amplitude of the received UWB signals

### BRIEF DESCRIPTION OF THE DRAWINGS

The above and other objects, features and advantages of the present disclosure will be more apparent from the following detailed description taken in conjunction with the accompanying drawings.
FIG. 1 is a diagram showing a configuration of a driver monitoring system according to an exemplary embodiment of the present inventive concept.
FIG. 2 is a block diagram showing a configuration of a heartbeat measuring apparatus according to an exemplary embodiment of the present inventive concept.
FIG. 3 is a block diagram showing a detailed configuration of a measurer according to an exemplary embodiment of the present inventive concept.
FIGS. 4A and 4B are diagrams for describing a principle of detecting a signal according to an exemplary embodiment of the present inventive concept.
FIG. 5 is a diagram for describing a change in a center frequency of a signal penetrating a heart of a subject.
FIG. 6 is a diagram for describing an amplitude change of a signal penetrating a heart of the subject.
FIG. 7 is a flow chart for describing a heartbeat measuring method according to an exemplary embodiment of the present inventive concept.

### DETAILED DESCRIPTION

Hereinafter, exemplary embodiments of the present inventive concept will be described in detail with reference to the accompanying drawings.

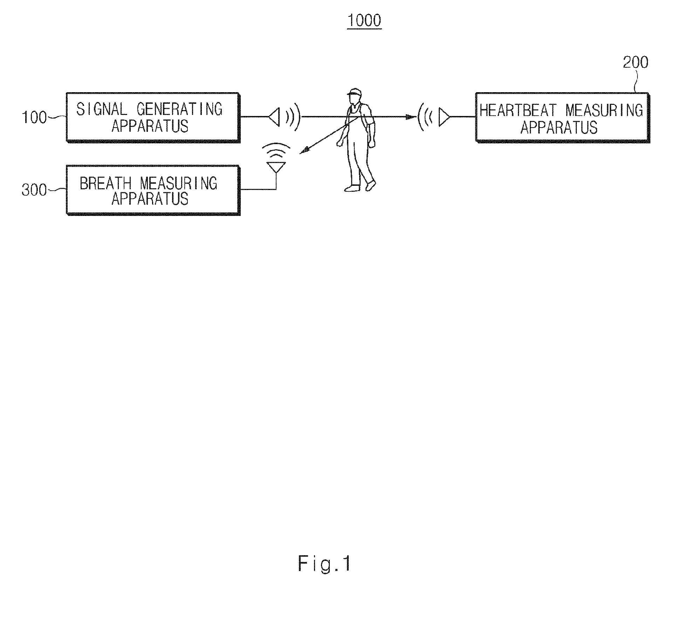

FIG. 1 is a diagram showing a configuration of a driver monitoring system according to an exemplary embodiment of the present inventive concept.

Referring to FIG. 1, a driver monitoring system 1000 may include a signal generating apparatus 100, a heartbeat measuring apparatus 200, and a breath measuring apparatus 300.

The signal generating apparatus 100 may generate and transmit a signal. The signal generating apparatus 100 may generate a signal having a predetermined signal property (e.g., center frequency, amplitude, or the like) and continuously emit the signal to the surroundings. For example, the signal generating apparatus 100 may transmit the signal at periods of 1 to 5ms. Particularly, the signal generating apparatus 100 may generate an ultra wideband (UWB) signal.

Meanwhile, the signal generating apparatus 100 may be implemented in a form embedded in a steering wheel of a vehicle or a driver's seat sheet.

The heartbeat measuring apparatus 200 may receive a signal penetrating a subject and measure a heartbeat of the subject. Specifically, the heartbeat measuring apparatus 200 may receive the signal penetrating the subject and measure the heartbeat of the subject using a variation of at least one of center frequency and amplitude of the received signal.

The heartbeat measuring apparatus 200 may be positioned in a direction opposite to the signal generating apparatus 100 based on the subject. For example, the heartbeat measuring apparatus 200 may be positioned opposite to the signal generating apparatus 100 with respect to the subject. The reason is that the heartbeat measuring apparatus 200 may measure the heartbeat of the driver using the signal penetrating the heart of the driver. For example, when the signal generating apparatus 100 is positioned in the steering wheel, the heartbeat measuring apparatus 200 may be implemented in a form embedded in the driver's seat sheet. As another example, when the signal generating apparatus 100 is positioned in the driver's seat sheet, the heartbeat measuring apparatus 200 may be implemented in a form embedded in the steering wheel or the driver's seat sheet. As another example, among the configurations of the signal generating apparatus 100 and the heartbeat measuring apparatus 200, only the configuration of transmitting the signal and the configuration of receiving the signal may be positioned in a direction opposite to each other (e.g., positioned opposite to each other with respect to the subject). In this case, the remaining configurations of the signal generating apparatus 100 and the heartbeat measuring apparatus 200 may be implemented in a physically single form.

The breath measuring apparatus 300 may receive a signal reflected from the subject and measure breath or motion of the subject. The breath measuring apparatus 300 may measure the breath or the motion of the subject using a delayed amount of time of the signal reflected from the subject. The breath measuring apparatus 300 may compare a time of receiving the reflected signal with a time of transmitting the signal from the signal generating apparatus 100 to measure the delayed amount of time. The breath measuring apparatus 300 may be connected to the signal generating apparatus 100 to measure the delayed amount of time and may receive the time at which the signal is transmitted from the signal generating apparatus 100. In addition, the breath measuring apparatus 300 may measure the breath or the motion of the subject by monitoring the delayed amount of time.

That is, when the subject breathes in, a chest of subject is expanded, such that a distance, in which the signal transmitted from the signal generating apparatus 100 is reflected from the subject and is then received by the breath measuring apparatus 300, becomes short. As a result, the delayed amount of time of the received signal may be decreased. In addition, when the subject breathes out, the chest of subject is contracted, such that the distance, in which the signal transmitted from the signal generating apparatus 100 is reflected from the subject and is then received by the breath measuring apparatus 300, becomes long. As a result, the delayed amount of time of the received signal may be increased. The breath measuring apparatus 300 may measure the breath or the motion of the subject by continuously monitoring the delayed amount of time of the received signal.

Meanwhile, the breath measuring apparatus 300 among the configurations of the driver monitoring system 1000 may be omitted depending on an exemplary embodiment of the present inventive concept.

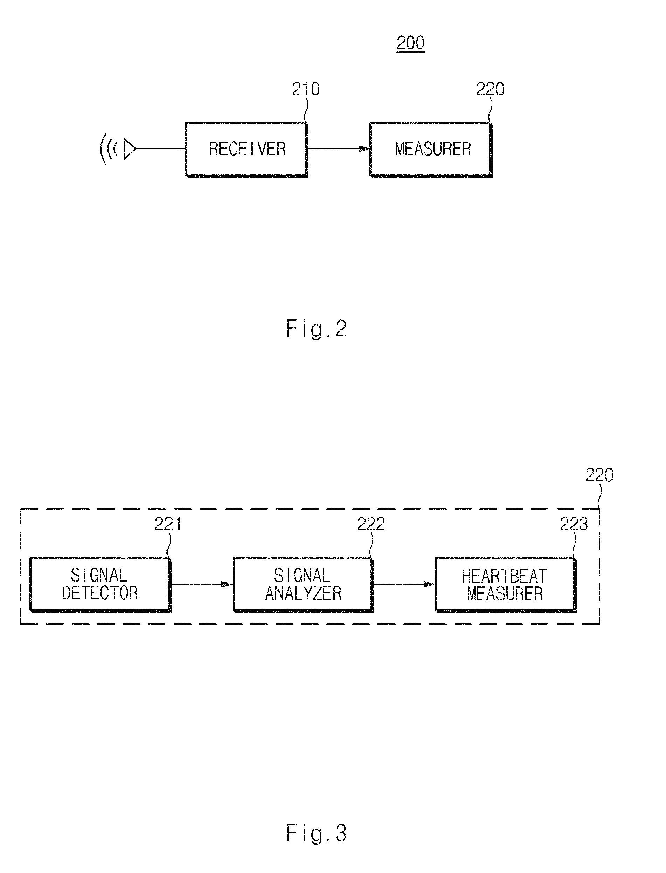

FIG. 2 is a block diagram showing a configuration of a heartbeat measuring apparatus according to an exemplary embodiment of the present inventive concept.

Referring to FIG. 2, the heartbeat measuring apparatus 200 may include a receiver 210 and a measurer 220.

The receiver 210 may receive a signal penetrating the subject among signals transmitted from the signal generating apparatus 100. The receiver 210 may include a low-noise amplifier for amplifying the received signal.

The measurer 220 may measure the heartbeat of the subject using variation of at least one of center frequency and amplitude of the signal received by the receiver 210. The measurer 220 will be described in detail with reference to FIGS. 3 to 6.

FIG. 3 is a block diagram showing a detailed configuration of a measurer according to an exemplary embodiment of the present inventive concept.

Referring to FIG. 3, the measurer 220 may include a signal detector 221, a signal analyzer 222, and a heartbeat measurer 223.

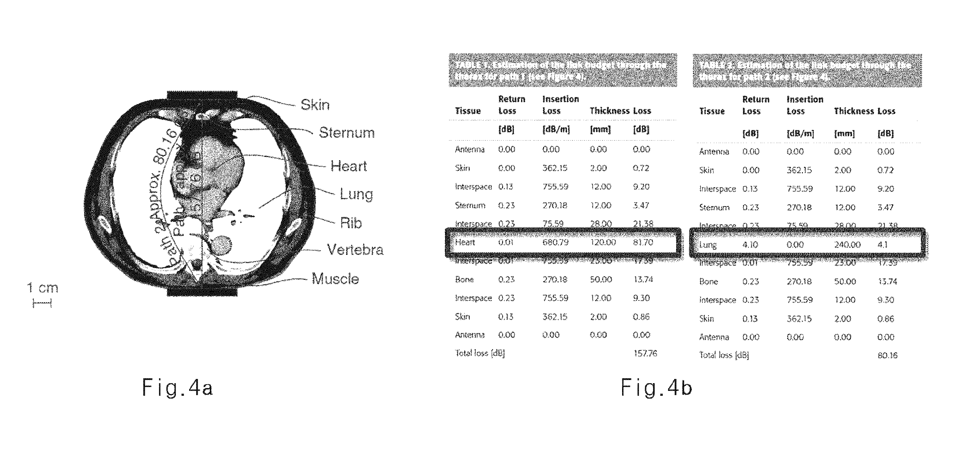

The signal detector 221 may detect a signal penetrating the heart of the subject among the signals received by the receiver 210. As an example of detecting the signal, a method using an attenuation amount of the signal will be described with reference to FIGS. 4A and 4B.

FIGS. 4A and 4B are diagrams for describing a principle of detecting a signal according to an exemplary embodiment of the present inventive concept.

Referring to FIG. 4A, when the signal penetrates the body, it may penetrate the body through two paths. A first path (path 1) is a path penetrating the heart and a second path (path 2) is a path penetrating lungs. Because the heart is filled with liquid and the lungs are filled with gas, attenuation characteristics of the signal may be different when the signal penetrates two paths.

Tables in FIG. 4B show attenuation characteristics of the signal when the signal penetrates the first path and the second path, respectively. Referring to FIG. 4B, it may be appreciated that a (signal) loss in the first path (through the heart) is 157.76dB and a (signal) loss in the second path (through the lung) is 80.16dB. That is, the path penetrating the heart and the path penetrating the lungs have very different signal attenuation degrees. Therefore, the signal detector 221 may classify the received signal into two groups depending on strength of the signal and may detect the group having a small strength of the signal as the signal penetrating the heart.

As another example of detecting the signal, time receiving the signal may be used. Specifically, the signal detector 221 may detect an initially received signal among signals transmitted from the signal generating apparatus 100 at the same time. The initially received signal among the signals transmitted from the signal generating apparatus 100 may correspond to a signal having the shortest transmitting distance. That is, the initially received signal may correspond to a signal rectilinearly passing between the signal generating apparatus 100 and the heartbeat measuring apparatus 200. Referring to FIG. 4A. That is, the signal rectilinearly passing between the signal generating apparatus 100 and the heartbeat measuring apparatus 200 passes through the heart of the subject.

The signal analyzer 222 may analyzes the signal detected by the signal detector 221. According to the exemplary embodiment of the present inventive concept, the signal analyzer 222 may analyze the signal in two ways.

According to a first exemplary embodiment of the present inventive concept, the signal analyzer 222 may analyze variation of at least one of center frequency and amplitude of the detected signal. The signal generating apparatus 100 may periodically transmit a signal having predetermined signal characteristics. In addition, the signal analyzer 222 may recognize the center frequency and amplitude of the signal transmitted from the signal generating apparatus 100 in advance, and may compares the center frequency and amplitude with center frequency and amplitude of the received signal to thereby analyze the variation of the center frequency and amplitude. When the UWB signal penetrates the heart of the subject, the center frequency and amplitude of the UWB signal may be decreased.

According to a second exemplary embodiment of the present inventive concept, the signal analyzer 222 may analyze at least one of the center frequency and amplitude of the detected signal. That is, unlike the first exemplary embodiment in which the variation of the received signal is analyzed, the center frequency and amplitude of the received signal may be simply measured.

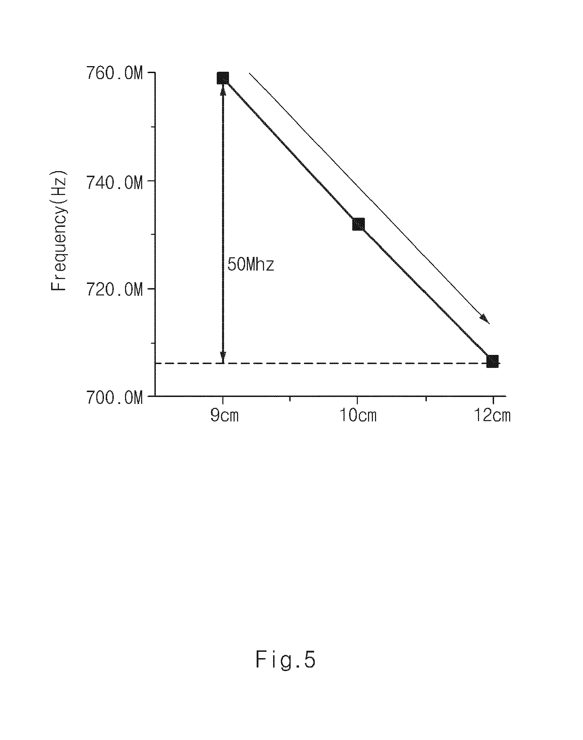

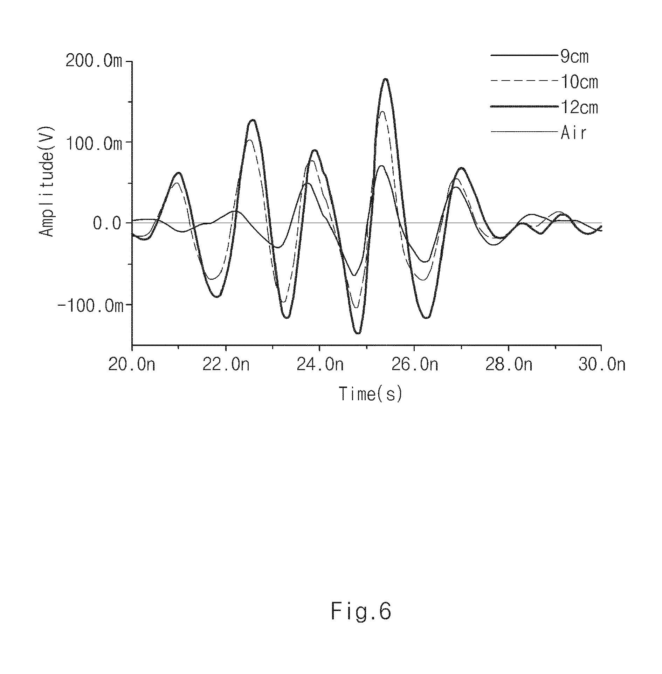

The heartbeat measurer 223 may measure the heartbeat of the subject using the analyzed result of the signal analyzer 222. Specifically, the heartbeat measurer 223 may measure the heartbeat of the subject by monitoring the analyzed result of the signal analyzer 222. A principle of measuring the heartbeat by the heartbeat measurer 223 will be described with reference to FIGS. 5 and 6.

FIG. 5 is a diagram for describing a change in a center frequency of a signal penetrating a heart of a subject.

As the signal transmitted from the signal generating apparatus 100 penetrates the heart of the subject, the center frequency thereof may be decreased. In addition, a decreased amount of center frequency may be varied depending on a size of the heart.

A relationship between the size of the heart and the decreased amount of center frequency may be confirmed from the FIG. 5. FIG. 5 corresponds to a result generated by performing an experiment on a water balloon in order to make an environment similar to the heart. Referring to FIG. 5, it may be appreciated that the center frequency of the signal passing through the water balloon is decreased and as the size of the water balloon is increased, the decreased amount of center frequency is increased.

The heart of the subject may be repeatedly contracted and relaxed. When being contracted, the size of the heart may be decreased, and when being relaxed, the size of the heart may be increased. That is, the heartbeat measurer 223 may determine whether or not the heart is contracted or relaxed by monitoring the center frequency of the received signal or the variation of the center frequency.

FIG. 6 is a diagram for describing an amplitude change of the signal penetrating the heart of the subject.

As the signal transmitted from the signal generating apparatus 100 penetrates the heart of the subject, the amplitude thereof may be decreased. In addition, a decreased amount of amplitude may be varied depending on the size of the heart.

The decreased amount of amplitude according to the size of the heart may be confirmed from FIG. 6. Similar to FIG. 5, FIG. 6 corresponds to a result generated by performing an experiment on a water balloon in order to make an environment similar to the heart. It may be appreciated from FIG. 6 that as the size of the water balloon is increased, the decreased amount of amplitude is increased.

The heart of the subject may be repeatedly contracted and relaxed. When being contracted, the size of the heart may be decreased, and when being relaxed, the size of the heart may be increased. That is, the heartbeat measurer 223 may determine whether or not the heart is contracted or relaxed by monitoring the amplitude of the received signal or the variation of the amplitude.

The heartbeat measuring apparatus according to an exemplary embodiment of the present inventive concept may measure the heartbeat regardless of a location of the heart unlike a heartbeat measuring apparatus based on a reflective wave. Therefore, even though the subject moves during the heartbeat measurement, when the heart of the subject is located in a penetration path of the signal, the heartbeat may be measured.

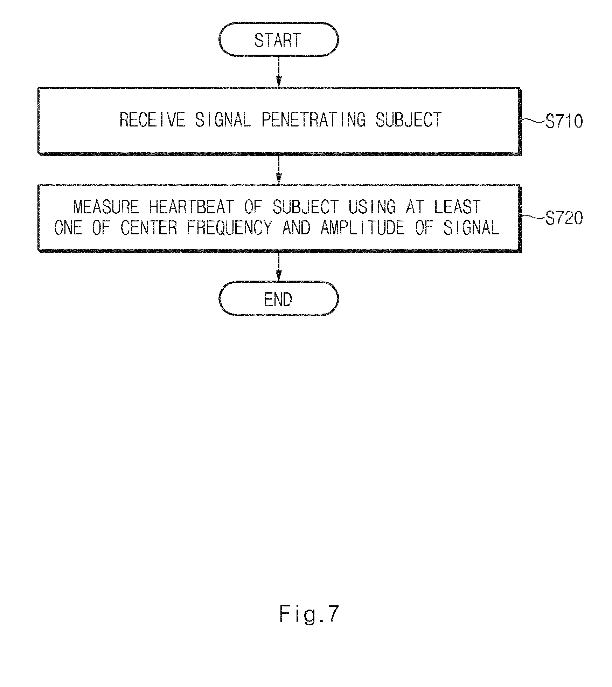

FIG. 7 is a flow chart for describing a heartbeat measuring method according to an exemplary embodiment of the present inventive concept.

Referring to FIG. 7, the heartbeat measuring apparatus 200 may firstly receive the signal penetrating the subject (S710). Here, the signal penetrating the subject may be an ultra wideband (UWB) signal.

In addition, the heartbeat measuring apparatus 200 may measure the heartbeat of the subject using at least one of the center frequency and amplitude of the received signal (S720). Describing processes of measuring the heartbeat in detail, first, a signal penetrating the heart of the subject among the received signals may be detected. Here, when the signal is detected, the attenuation amount of signal may be used or the time receiving the signal may be used.

The path penetrating the heart and the path penetrating the lungs have very different signal attenuation degrees. Therefore, the heartbeat measuring apparatus 200 may classify the received signal into two groups depending on strength of the signal and may detect the group having a small strength of the signal as the signal penetrating the heart.

Alternatively, the heartbeat measuring apparatus 200 may detect an initially received signal among the received signals as the signal penetrating the heart. Because the initially received signal may be received through a straight line path, it may correspond to a signal having the shortest transmission distance. When it is configured such that the heart of the subject is positioned in the straight line path between the signal generating apparatus 100 and the heartbeat measuring apparatus 200, the initially received signal may be detected as the signal penetrating the heart.

In addition, at least one of the center frequency and amplitude of the detected signal may be analyzed. When the detected signal is analyzed, the center frequency and amplitude may be analyzed or the variation of the center frequency and amplitude may be analyzed.

In addition, the heartbeat of the subject may be measured by monitoring the analyzed result. When the UWB signal penetrating the heart is received, the center frequency and amplitude thereof may be decreased. In addition, as the size of the heart is increased, the decreased amount may be increased. As a result, a contraction or relaxation of the heart may be determined by continuously monitoring the analyzed result.

As described above, according to exemplary embodiments of the present inventive concept, the heartbeat may be measured regardless of the location of the heart. Therefore, even though the subject moves during the heartbeat measurement, when the heart of the subject is located in the penetration path of the signal, the heartbeat may be stably measured.

Further, because the breath measurement together with the heartbeat measurement is possible, the state of the subject may be more accurately observed.

Although some embodiments of the present inventive concept have been disclosed for illustrative purposes, those skilled in the art will appreciate that various modifications, additions and substitutions are possible, without departing from the scope and spirit of the disclosure as disclosed in the accompanying claims. Accordingly, such modifications, additions and substitutions should also be understood to fall within the scope of the present inventive concept.

### SYMBOL OF EACH OF THE ELEMENTS IN THE FIGURES

- 100:: SIGNAL GENERATING APPARATUS
- 200:: HEARTBEAT MEASURING APPARATUS
- 210:: RECEIVER
- 220:: MEASURER
- 221:: SIGNAL DETECTOR
- 222:: SIGNAL ANALYZER
- 223:: HEARTBEAT MEASURER
- 300:: BREATH MEASURING APPARATUS

## Claims

1. A heartbeat measuring apparatus, comprising:
a receiver configured to receive ultra wideband (UWB) signals penetrating a subject; and
a measurer configured to measure a heartbeat of the subject using at least one of center frequency and amplitude of the received UWB signals.

2. The heartbeat measuring apparatus according to claim 1, wherein the measurer includes:
a signal detector configured to detect a UWB signal penetrating a heart of the subject among the received UWB signals;
a signal analyzer configured to analyze a variation of at least one of center frequency and amplitude of the detected UWB signal; and
a heartbeat measurer configured to measure the heartbeat of the subject by monitoring the variation.

3. The heartbeat measuring apparatus according to claim 1, wherein the measurer includes:
a signal detector configured to detect the UWB signal penetrating a heart of the subject among the received UWB signals;
a signal analyzer configured to analyze at least one of center frequency and amplitude of the detected UWB signal; and
a heartbeat measurer configured to measure the heartbeat of the subject by monitoring the at least one of center frequency and amplitude of the detected UWB signal.

4. A driver monitoring system, comprising:
a signal generating apparatus configured to generate and transmit an ultra wideband (UWB) signal; and
a heartbeat measuring apparatus configured to receive the UVB signal penetrating a subject and measure a heartbeat of the subject using at least one of center frequency and amplitude of the received UWB signal.

5. The driver monitoring system according to claim 4, wherein the heartbeat measuring apparatus is positioned opposite to the signal generating apparatus with respect to the subject.

6. The driver monitoring system according to claim 4, further comprising a breath measuring apparatus configured to receive the UWB signal reflected from the subject and measure a breath or a motion of the subject using a delayed amount of time of the received UWB signal.

7. A heartbeat measuring method, comprising:
receiving ultra wideband (UWB) signals penetrating a subject; and
measuring a heartbeat of the subject using at least one of center frequency and amplitude of the received UWB signals.

8. The heartbeat measuring method according to claim 7, wherein the measuring of the heartbeat includes:
detecting a UWB signal penetrating a heart of the subject among the received UWB signals;
analyzing a variation of at least one of center frequency and amplitude of the detected UWB signal; and
measuring the heartbeat of the subject by monitoring the variation.

9. The heartbeat measuring method according to claim 7, wherein the measuring of the heartbeat includes:
detecting a UWB signal penetrating a heart of the subject among the received UWB signals;
analyzing at least one of center frequency and amplitude of the detected UWB signal; and
measuring the heartbeat of the subject by monitoring the at least one of center frequency and amplitude of the detected UWB signal.

10. The heartbeat measuring apparatus according to claim 1, wherein the measurer is configured to determine whether or not a heart of the subject is contracted or relaxed by monitoring the at least one of center frequency and amplitude of the received UWB signals.

11. The driver monitoring system according to claim 4, wherein the heartbeat measuring apparatus is configured to determine whether or not the heart of the subject is contracted or relaxed by monitoring the at least one of center frequency and amplitude of the received UWB signal.

12. The heartbeat measuring method according to claim 7, wherein the measuring of the heartbeat of the subject includes determining whether or not a heart of the subject is contracted or relaxed by monitoring the at least one of center frequency and amplitude of the received UWB signals.
